(19) Europäisches Patentamt
European Patent Office
Office européen des brevets

(11) Veröffentlichungsnummer: **0 205 035**
**A2**

(12) **EUROPÄISCHE PATENTANMELDUNG**

(21) Anmeldenummer: 86107213.0

(22) Anmeldetag: 28.05.86

(51) Int. Cl.4: C 07 C 45/49
//C07C47/06

(30) Priorität: 10.06.85 DE 3520689

(43) Veröffentlichungstag der Anmeldung:
17.12.86 Patentblatt 86/51

(84) Benannte Vertragsstaaten:
AT BE CH DE FR GB IT LI LU NL SE

(71) Anmelder: Union Rheinische Braunkohlen Kraftstoff Aktiengesellschaft
Ludwigshafener Strasse o. Nr. Postfach 8
D-5047 Wesseling(DE)

(72) Erfinder: Korff, Joachim, Dr.
Kapellenstrasse 28
D-5303 Bornheim-Merten(DE)

(72) Erfinder: Keim, Karl-Heinz, Dipl.-Ing.
Höhenring 31
D-5357 Swisttal-Heimerzheim(DE)

(54) **Verbessertes Verfahren zur Homologisierung von Methanol.**

(57) Die Erfindung betrifft ein katalytisches Verfahren zur Homologisierung von Methanol unter möglichst vollständigem Ausschluß von Eisen und/oder Eisenverbindungen.

Fig. 1

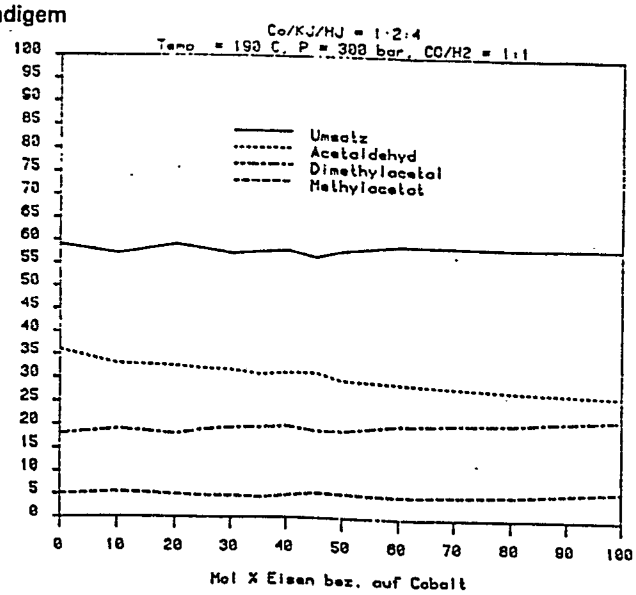


EP 0 205 035 A2

# Verbessertes Verfahren zur Homologisierung von Methanol

Die Erfindung betrifft ein katalytisches Verfahren zur Homologisierung von Methanol unter möglichst vollständigem Ausschluß von Eisen und/oder Eisenverbindungen.

Die Homologisierung von Methanol zu Sauerstoff enthaltenden $C_2$-Verbindungen ist in zahlreichen Veröffentlichungen beschrieben worden. Als Katalysatoren werden insbesondere Kobalt und Kobalt-Verbindungen gemeinsam mit zusätzlichen Katalysatorkomponenten verwendet.
Neben dem Zusatz von Jod- und Bromverbindungen sind außer Kobalt auch andere Metalle als Katalysatorkomponenten beschrieben worden. Hierzu gehören auch Eisen und seine Verbindungen.
In US-PS 4 126 752 wird die Umsetzung von Methanol mit Kohlenmonoxid zu Ethanol in Gegenwart von Wasser beschrieben, wobei als Metall-Katalysatoren Kobalt-, Ruthenium-, Rhodium- oder Eisensalze offenbart sind.
In EPA 00 27 000 wird die Homologisierung von Methanol zu Methylacetat, Ethanol, Acetaldehyd oder deren Gemischen beschrieben, wobei Eisen-Kobalt-Carbonylclusterkomplexe als Katalysatoren verwendet werden.
In EPA 00 30 110 wird die Synthese von Methanol, Ethanol und Essigsäure aus Synthesegas in Gegenwart von Rhodium und Zirkonium auf einem Träger beschrieben mit den Kokatalysatoren: Eisen, Mangan, Molybdän, Wolfram, Ruthenium, Chrom, Uran und Thorium.

Von Argonne National Laboratories wird in Journal of Molecular Catalysis, 17, 1982, S. 331-37 die Verwendung von Fe/Aminkomplexen für die Homologisierung beschrieben.

Obgleich die im Stand der Technik beschriebenen Umsetzungen wahrscheinlich nicht nach den gleichen Reaktionswegen ablaufen, ist festzustellen, daß der Fachmann Eisen als eine geeignete Katalysatorkomponente ansieht für Homologisierungen und verwandte Reaktionen.
Die Untersuchungen der Anmelderin haben nunmehr im Gegensatz zum Stand der Technik überraschend ergeben, daß die Gegenwart von Eisen und/oder dessen Verbindungen die katalytische Homologisierung von Methanol hinsichtlich des Gesamtumsatzes und hinsichtlich der Selektivität der Acetaldehydbildung nachteilig beeinflußt. Die vorliegende Erfindung betrifft daher ein Verfahren zur Homologisierung von Methanol durch katalytische Umsetzung von Methanol mit $H_2$ und CO bzw. mit $H_2$ und CO enthaltenden Gasen, dadurch gekennzeichnet, daß die Homologisierung unter möglichst vollständigem Ausschluß von Eisen und/oder dessen Verbindungen durchgeführt wird.

Hierbei ist es unwesentlich, auf welchem Wege Eisen und/oder seine Verbindungen in die Homologisierungsreaktion gelangen, ob dies beispielsweise gemäß dem bisherigen Stand der Technik als Katalysatorbestandteil geschieht oder über die Bildung von Eisencarbonylen aus Eisen enthaltenden apparativen Teilen der Homologisierungsanlage, wie z.B. aus Zuführungsleitungen für CO oder Synthesegas oder beispielsweise Eisen enthaltenden Reaktorteilen.
Die nachteilige Wirkung von Eisen und/oder dessen Verbindungen tritt sowohl in Gegenwart von Lösungsmitteln, als auch ohne Lösungsmittel ein. Der Einfluß des Eisens ist in den folgenden Figuren dargestellt:

<u>Fig. 1</u>

stellt den Umsatz des eingesetzten Methanols und die Selektivitäten bezüglich Acetaldehyd, Acetaldehyd-Dimethylacetal sowie

Essigsäuremethylester dar ohne Lösungsmittelzusatz mit Co/KJ/HJ als Katalysator.

Fig. 2

entspricht Fig. 1, jedoch ist Dioxan als Lösungsmittel im Verhältnis Dioxan : Methanol = 1 : 1 enthalten.

Fig. 3

stellt den Umsatz des eingesetzten Methanols und die Selektivität bezüglich der Produkte dar mit CO/HJ und Triphenylphosphin als Katalysator.

Fig. 4

entspricht Fig. 3, jedoch ist Dioxan als Lösungsmittel im Verhältnis Dioxan : Methanol = 1 : 1 enthalten.

Fig. 5

stellt die Zunahme der Eisenkonzentration mit der Anzahl der Rückführungen dar, in Mol. % Eisen pro Mol. % Kobalt.

Die in den Figuren dargestellten Versuche wurden kontinuierlich bei typischen Homologisierungsbedingungen durchgeführt, nämlich bei Temperaturen von 190 - 200 °C, einem Druck von 300 bar, einem $CO/H_2$-Molverhältnis von 1 : 1 und einer LHSV von 500 ml/h. Das erfindungsgemäße Verfahren betrifft jedoch auch sonstige übliche Reaktionsbedingungen, wie Temperaturen von 140 - 300 °C, Drücke von 50 - 600 bar, $CO/H_2$-Verhältnisse von 10 : 1 bis 1 : 10 und Verweilzeiten bis max. 10 Stunden.

Das erfindungsgemäße Verfahren betrifft insbesondere die Kobalt enthaltenden Katalysatoren, jedoch auch die sonstigen Homologisierungskatalysatoren wie beispielsweise solche, die Ni, Ru, Os, Pt, Mo, W, Mn, Cr, U, Th, La, Sc, Y, Re u.a. enthalten sowie diejenigen, die Gemische der genannten Metalle enthalten.

Es ist bekannt, daß Kobalt enthaltende Katalysatoren, aber auch Ruthenium enthaltende Katalysatoren von besonderer Bedeutung bei der Homologisierung sind, wobei Kobalt als fein verteiltes metallisches Kobalt, aber auch in Form von Kobaltverbindungen zugesetzt werden kann.
In zahlreichen im Stand der Technik beschriebenen Fällen enthält das Katalysatorsystem zusätzlich Halogenverbindungen wie Jodide, Bromide und Chloride, jedoch bevorzugt Jodide. Diese können sowohl salzartig als auch kovalent vorliegen, wobei auch salzartige und kovalente Halogenide nebeneinander vorliegen können. Hierbei kann sowohl das salzartige als auch das homöopolar gebundene Halogenid im Überschuß vorliegen.
Neben Kobalt können die oben genannten weiteren Metalle auch als Kokatalysatoren vorliegen.

Ferner können Liganden der allgemeinen Formel im Katalysatorsystem vorliegen:

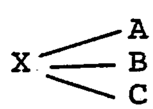

wobei X Phosphor, Stickstoff, Arsen, Antimon oder Wismuth sein kann, während A, B und C Wasserstoff, Alkylgruppen, Alkoxygruppen, aromatische Gruppen, Aryloxygruppen, Cycloalkylgruppen u. a. sein können.
Gleiches gilt für Liganden der Struktur:

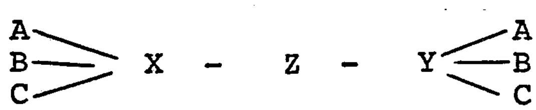

wobei X und Y = P, N, As, Sb oder Bi sein können und Z unverzweigte oder verzweigte und substituierte Methylengruppen sein können, die auch Sauerstoff und andere Heteroatome in der Kette enthalten können. Die Liganden können beispielsweise auch in Form von Phosphonium, Ammonium-, Arsoniumverbindungen, insbesondere Halogeniden vorliegen.

Die genannten Katalysatorkomponenten sind jedoch nicht als einschränkend anzusehen, da die erfindungsgemäße Wirkung des Eisens im wesentlichen unabhängig von der Art der Komponenten ist.
Die vorliegende Erfindung ist auch im wesentlichen unabhängig von den Verhältnissen der Katalysatorkomponenten untereinander sowie der Variation sonstiger Parameter, wie Druck, Temperatur, $CO/H_2$-Verhältnis, Verweilzeit, Co- bzw. Metall/Halogenverhältnis, Co- bzw. Metall/Ligandenverhältnis, Co- bzw. Metall/Methanolverhältnis, Co/Kokatalysator-Verhältnis, Liganden/Halogenidverhältnis und Verhältnis kovalenten Halogenids zu salzartigem Halogenid u.a.
Es wurde erfindungsgemäß gefunden, daß hierbei nur graduelle Unterschiede auftreten, daß sich also Umsatz und Selektivität der Homologisierung in gewissem Umfang unterschiedlich schnell verschlechtern, daß jedoch die Gegenwart des Eisens generell, in Abhängigkeit von der Umsetzungsdauer, unabhängig davon, ob die Umsetzung hierbei diskontinuierlich oder kontinuierlich abläuft, zu einer Herabsetzung von Umsatz und Selektivität führt.

Das erfindungsgemäße Verfahren betrifft auch Homologisierungsreaktionen, die in Gegenwart von Lösungsmitteln durchgeführt werden, wobei das Verhältnis Lösungsmittel : Methanol entsprechend dem Stand der Technik in weiten Grenzen variieren kann. Beispielsweise kann das Lösungsmittel/Methanol-Verhältnis 0,2 bis 200 betragen. Als Lösungsmittel und sonstige Zusätze seien

beispielhaft genannt: Dioxane, Tetrahydrofurane, Carbonsäuren, Ester, Benzol, Alkoxy- und Aryloxyaromaten, Halogenbenzole, Polyethylenglykolether, Tetramethylensulfon, Alkane, Alkylaromaten, Dialkylether, Diphenylether, Siloxane u.a.

In den in den Figuren dargestellten Versuchsbeispielen betrug das Co/KJ/HJ-Molverhältnis 1 : 2 : 4, das $Co/P(Ph)_3/HJ$-Molverhältnis 1 : 2 : 6, das Co/MeOH-Verhältnis 1 : 2000 und im Falle des Einsatzes von Dioxan als Lösungsmittel, das Methanol/Dioxan-Verhältnis 1 : 1 Volumenteile.

Die Versuche wurden in einem Blasensäulenreaktor durchgeführt, die erfindungsgemäßen Versuche sind jedoch unabhängig von dem Reaktortyp. Die Aufarbeitung der Reaktionsprodukte kann nach einem der für die Aufarbeitung von Homologisierungsprodukten bekannten Verfahren erfolgen, wobei zu beachten ist, daß erfindungsgemäß im kontinuierlichen Betrieb der rückgeführte und wieder in die Homologisierung eingesetzte Katalysator möglichst frei von Eisen sein soll.

Da ein absoluter Ausschluß von Eisen bzw. Eisenverbindungen in technischen Anlagen kaum möglich ist, kann für den Fall, daß sich bei längeren Fahrperioden Eisen bzw. Eisenverbindungen akkumulieren, erfindungsgemäß durch Zusatz von heteropolar gebundenem Jodid und/oder kovalentem Jodid und/oder diese bildenden Verbindungen vorhandenes Eisen bzw. Eisenverbindungen inaktiviert werden. Die Ursache dieser Inaktivierung ist nicht bekannt, es ist jedoch möglich, daß insbesondere in Gegenwart von die Bildung von Komplexen begünstigenden Verbindungen wie z.B. von Lösungsmitteln wie Dioxan, eine Bindung des Eisens durch Komplexbildung erfolgt.
Eine weitere Methode, Verunreinigungen aus chemischen Umsetzungen auszuschleusen ist bekanntlich das teilweise Ausschleusen von verunreinigtem Katalysator bzw. das teilweise Regenerieren des ausgeschleusten Teils. Diese Maßnahme ist auch im Falle von Homologisierungsreaktionen anwendbar.
Anhand der Figuren wird die Erfindung näher erläutert.

Fig. 1 zeigt, daß die Umsatzkurve bei steigendem Eisengehalt nahezu horizontal verläuft. Da der Anteil an Acetalaldehyd-Dimethylacetal jedoch zunimmt, wobei pro Mol Acetaldehyd 2 Mol Methanol gebunden werden (die in der Umsatzkurve enthalten sind), nimmt der Umsatz von eingesetztem Methanol zu $C_2$-Produkten insgesamt ab.
Die Acetaldehydkurve zeigt mit zunehmendem Eisenanteil eine deutliche Abnahme.

Aus Fig. 2 geht hervor, daß in Gegenwart von Dioxan als Lösungsmittel ein nahezu gleicher Kurvenverlauf vorliegt, wobei jedoch die Absolutwerte des Umsatzes und der Selektivitäten nach oben verschoben sind.

Fig. 3 und 4 zeigen, daß auch bei Verwendung von Triphenylphosphin anstelle von Kaliumjodid analoge Umsatz-und Selektivitätsabnahmen erfolgen, wobei im Falle der Fig. 3 der Acetaldehyddimethylacetalanteil ebenfalls abnimmt, jedoch eine deutliche Zunahme höhere Produkte als $C_2$ zu beobachten ist.

Fig. 5 gibt den Anstieg der Konzentration des Eisens mit der Dauer des kontinuierlichen Betriebs bzw. der Anzahl der Rückführungen bei diskontinuierlichem Betrieb wieder.

Weitere Beispiele sind in der folgenden Tabelle angegeben, bei denen jeweils ein Versuch eisenfrei und 1 Vergleichsversuch (a) mit Zusatz von 0,3 Mol Eisen pro Mol Katalysatormetall durchgeführt wurden. Es wurden bei Zusatz von Fe vergleichbare Abnahmen von Umsatz und Selektivität gefunden, wie in den Figuren 1 bis 4 dargestellt.

Die Untersuchungen der Anmelderin haben gezeigt, daß bei eisenfreier Umsetzung, wobei alle anderen Parameter gleich sind, ein praktisch horizontaler Verlauf der Umsatz- und Selektivitätskurven vorliegt, auch bei wenigstens 2000-stündigem kontinuierlichem Betrieb.

Aus dem erfindungsgemäßen Ergebnis wird deutlich, daß entgegen dem bisherigen Kenntnisstand des Fachmanns eine erhebliche Verbesserung der Homologisierung von Methanol zu $C_2$-Verbindungen erzielt werden kann.

Tabelle

| Beispiel | Katalysator | T °C | P bar | Lösungsmittel | weitere Zusätze | Literatur |
|---|---|---|---|---|---|---|
| 1/1a | $Co(OAc)_2 \cdot 4\ H_2O$<br>$I_2$<br>$P(C_4H_9)_3$ | 200 | 200 | - | Chlorbenzol | EP-A 00 01 932 |
| 2/2a | $Co(OAc)_2 \cdot 4\ H_2O$<br>$PtCl_2$<br>LiJ | 195 | 270 | - | - | US-PS 4 389 532 |
| 3/3a | $Co(acetylacetonat)_2$<br>$As\ (C_6H_5)_3$<br>$J_2$ | 200 | 275 | - | - | US-PS 4 239 704 |
| 4/4a | $Co(acetylacetonat)_2$<br>$P\ (C_6H_5)_3\ /\ J_2$<br>$Ru(acetylacetonat)_2$ | 200 | 275 | - | - | US-PS 4 239 704 |
| 5/5a | Dicobaltoctacarbonyl<br>$KJ/CH_3J$<br>$J^-/Co = 102$<br>$CH_3J/J^- = 4{,}8$ | 200 | 250 | - | - | EP-A 00 11 042 |
| 6/6a | $Ni/Mo/P/Al_2O_3$<br>S | 250 | 172 | - | - | EP-A 00 99 665 |
| 7/7a | Ni/Mo/P<br>$SiO_2/Al_2O_3/H_2S$<br>behandelt | 240 | 82 | Dioxan | - | EP-A 00 99 665 |

## Verbessertes Verfahren zur Homologisierung von Methanol

### Patentansprüche

1. Verfahren zur Homologisierung von Methanol durch katalytische Umsetzung von Methanol mit $H_2$ und CO bzw. mit $H_2$ und CO enthaltenden Gasen, dadurch gekennzeichnet, daß die Homologisierung unter möglichst vollständigem Ausschluß von Eisen und/oder dessen Verbindungen durchgeführt wird.

2. Verfahren zur Homologisierung von Methanol durch katalytische Umsetzung von Methanol mit $H_2$ und CO bzw. mit $H_2$ und CO enthaltenden Gasen, dadurch gekennzeichnet, daß die Homologisierung unter möglichst vollständigem Ausschluß von Eisen und/oder dessen Verbindungen durchgeführt wird, wobei im Falle des Vorhandenseins kleiner Mengen an Eisen und/oder dessen Verbindungen durch Zusatz von heteropolar gebundenem Jod und/oder homöopolar gebundenem Jod und/oder durch Zusatz von heteropolar gebundenes Jod und/oder homöopolar gebundenes Jod bildenden Verbindungen, das Eisen und/oder dessen Verbindungen zumindest teilweise inaktiviert wird bzw. werden.

3. Verfahren nach den Ansprüchen 1 und 2, dadurch gekennzeichnet, daß der Homologisierungskatalysator Kobalt enthält.

4. Verfahren nach den Ansprüchen 1 bis 3, dadurch gekennzeichnet, daß der Katalysator Jod und/oder Jodverbindungen enthält.

Fig. 1

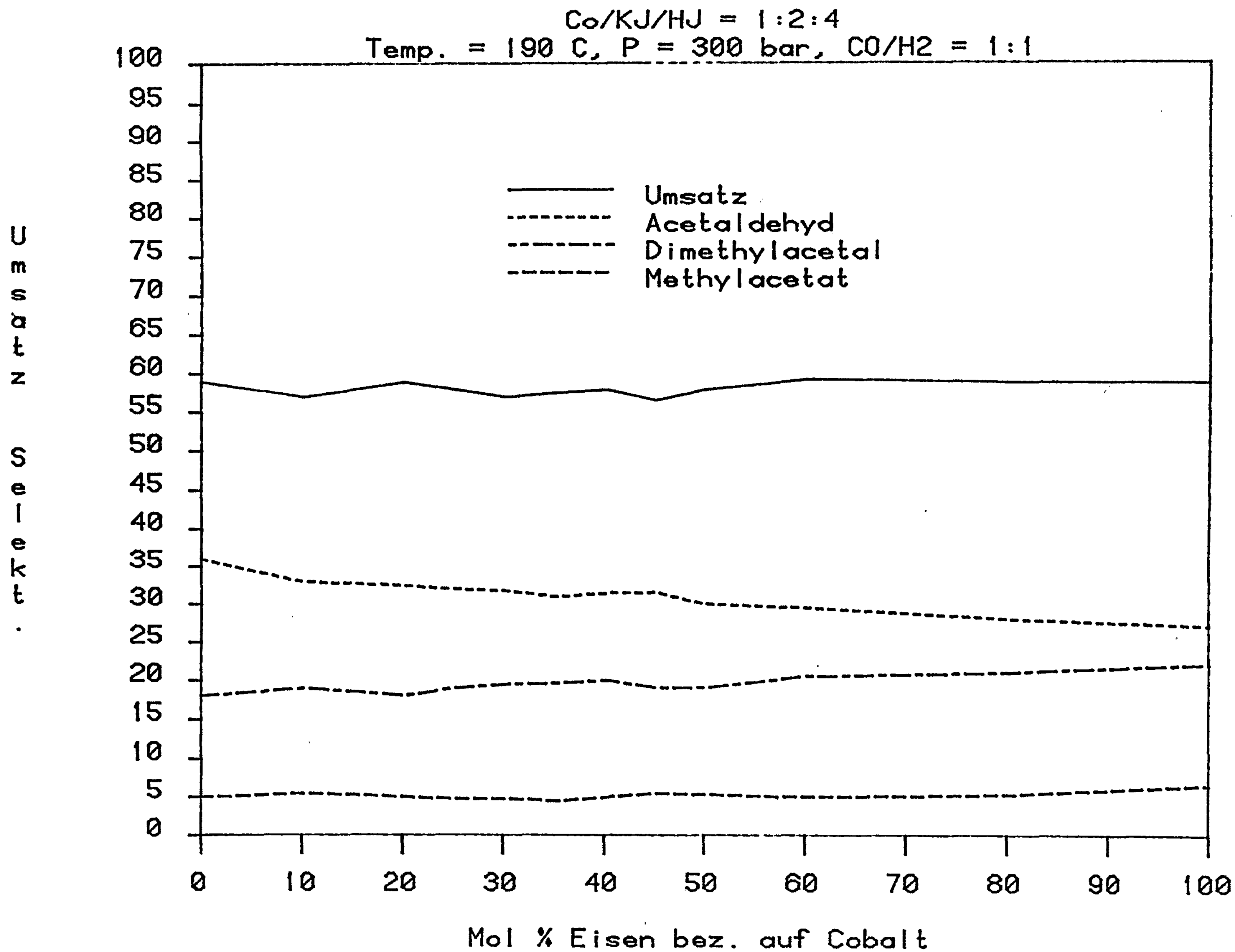
Co/KJ/HJ = 1:2:4
Temp. = 190 C, P = 300 bar, CO/H2 = 1:1
Umsatz
Acetaldehyd
Dimethylacetal
Methylacetat
Umsatz Selekt.
100
95
90
85
80
75
70
65
60
55
50
45
40
35
30
25
20
15
10
5
0
0
10
20
30
40
50
60
70
80
90
100
Mol % Eisen bez. auf Cobalt

Fig. 2

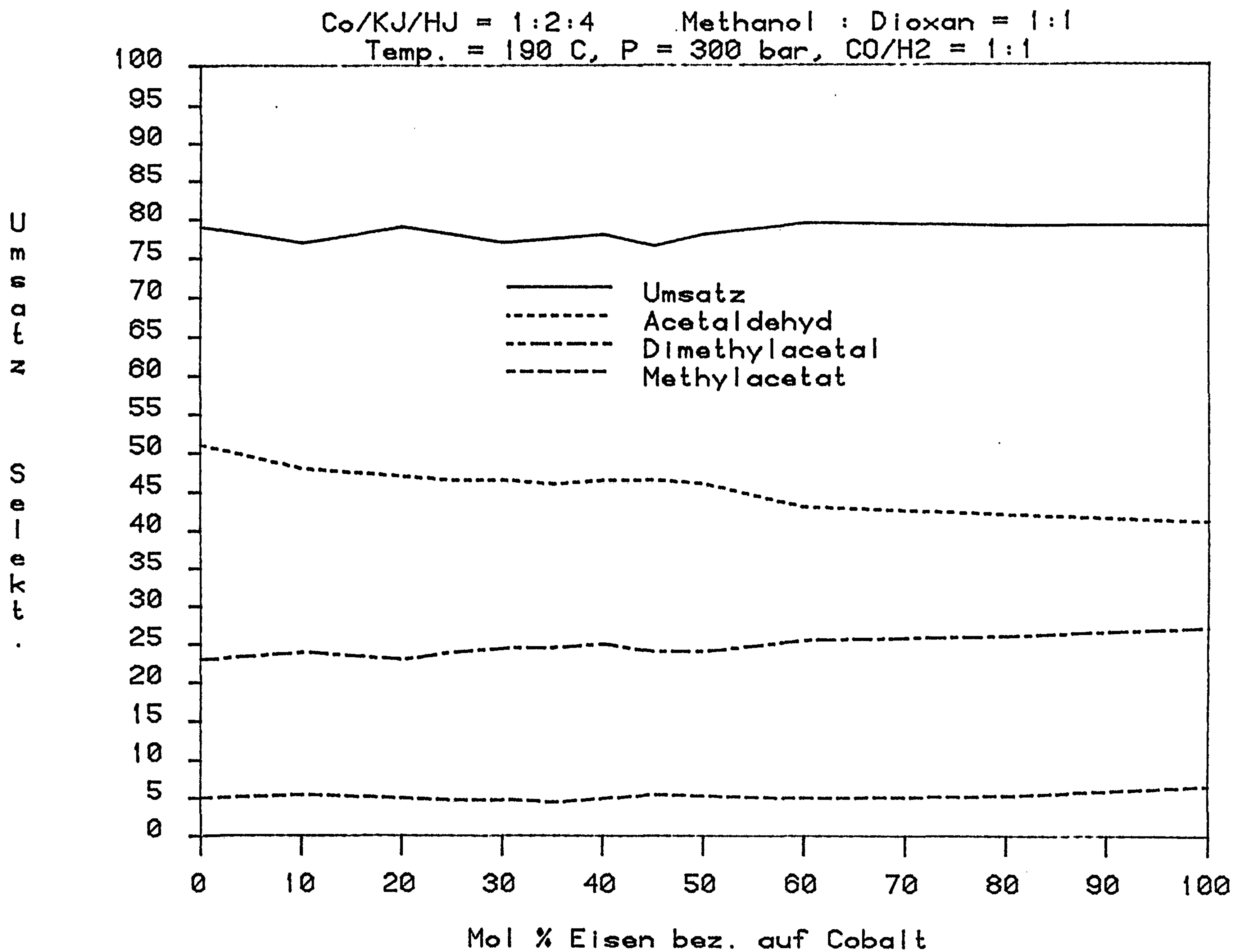

Co/KJ/HJ = 1:2:4 Methanol : Dioxan = 1:1
Temp. = 190 C, P = 300 bar, CO/H2 = 1:1
Umsatz
Acetaldehyd
Dimethylacetal
Methylacetat
Umsatz Selekt.
100 95 90 85 80 75 70 65 60 55 50 45 40 35 30 25 20 15 10 5 0
0 10 20 30 40 50 60 70 80 90 100
Mol % Eisen bez. auf Cobalt

3/5 0205035

Fig. 3

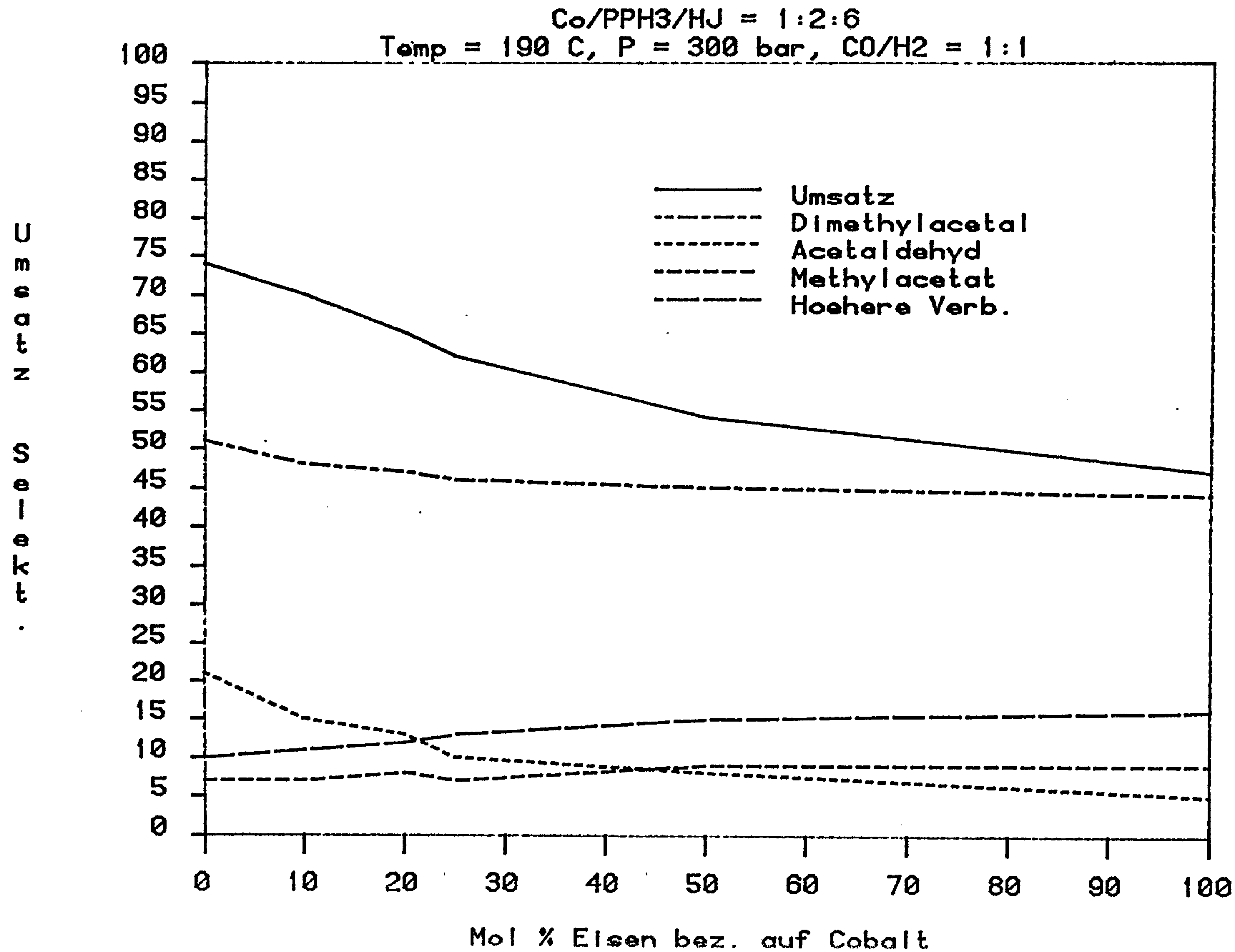

Fig. 4

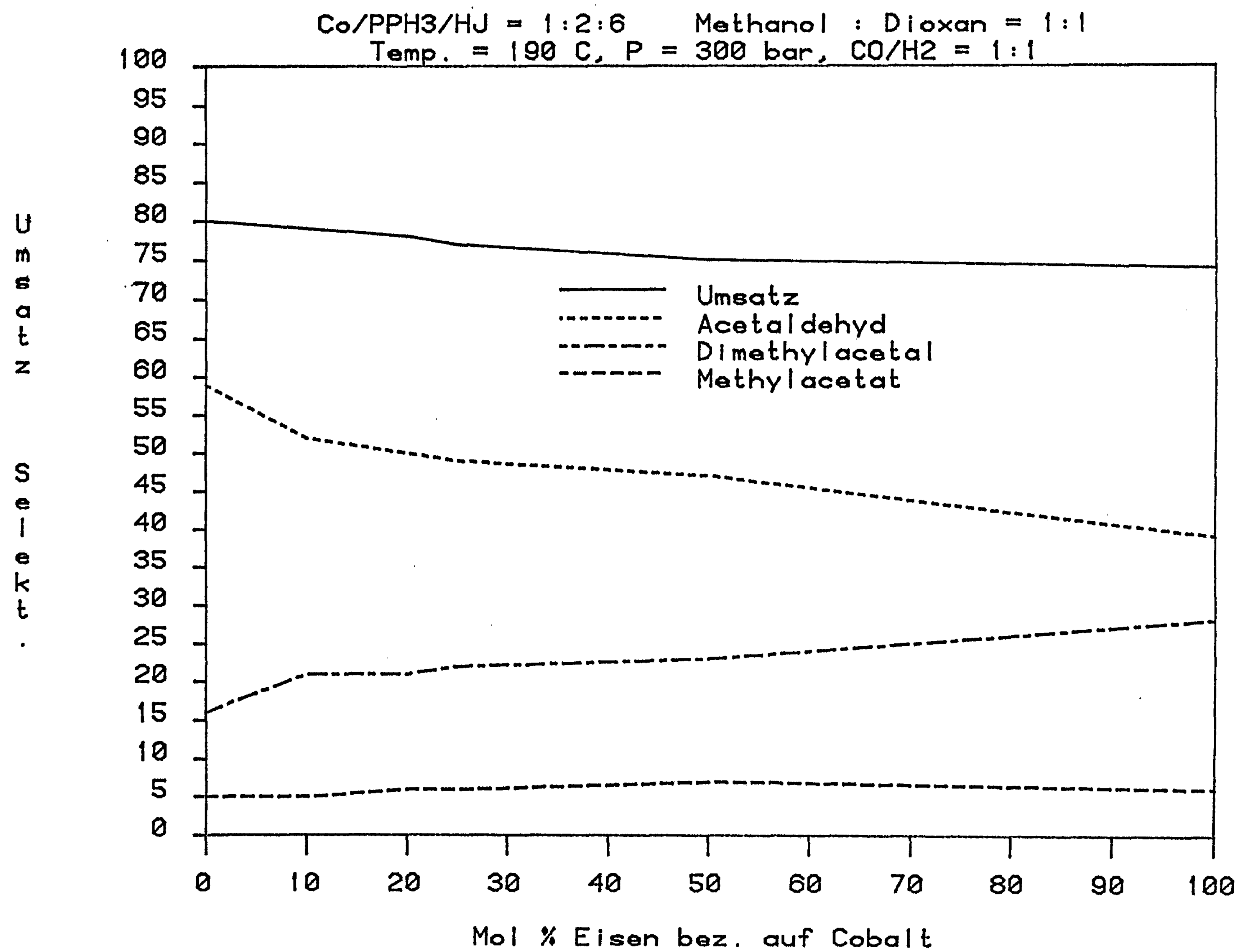
Co/PPH3/HJ = 1:2:6 Methanol : Dioxan = 1:1
Temp. = 190 C, P = 300 bar, CO/H2 = 1:1
Umsatz
Selekt.
100
95
90
85
80
75
70
65
60
55
50
45
40
35
30
25
20
15
10
5
0
Umsatz
Acetaldehyd
Dimethylacetal
Methylacetat
0
10
20
30
40
50
60
70
80
90
100
Mol % Eisen bez. auf Cobalt

Fig. 5

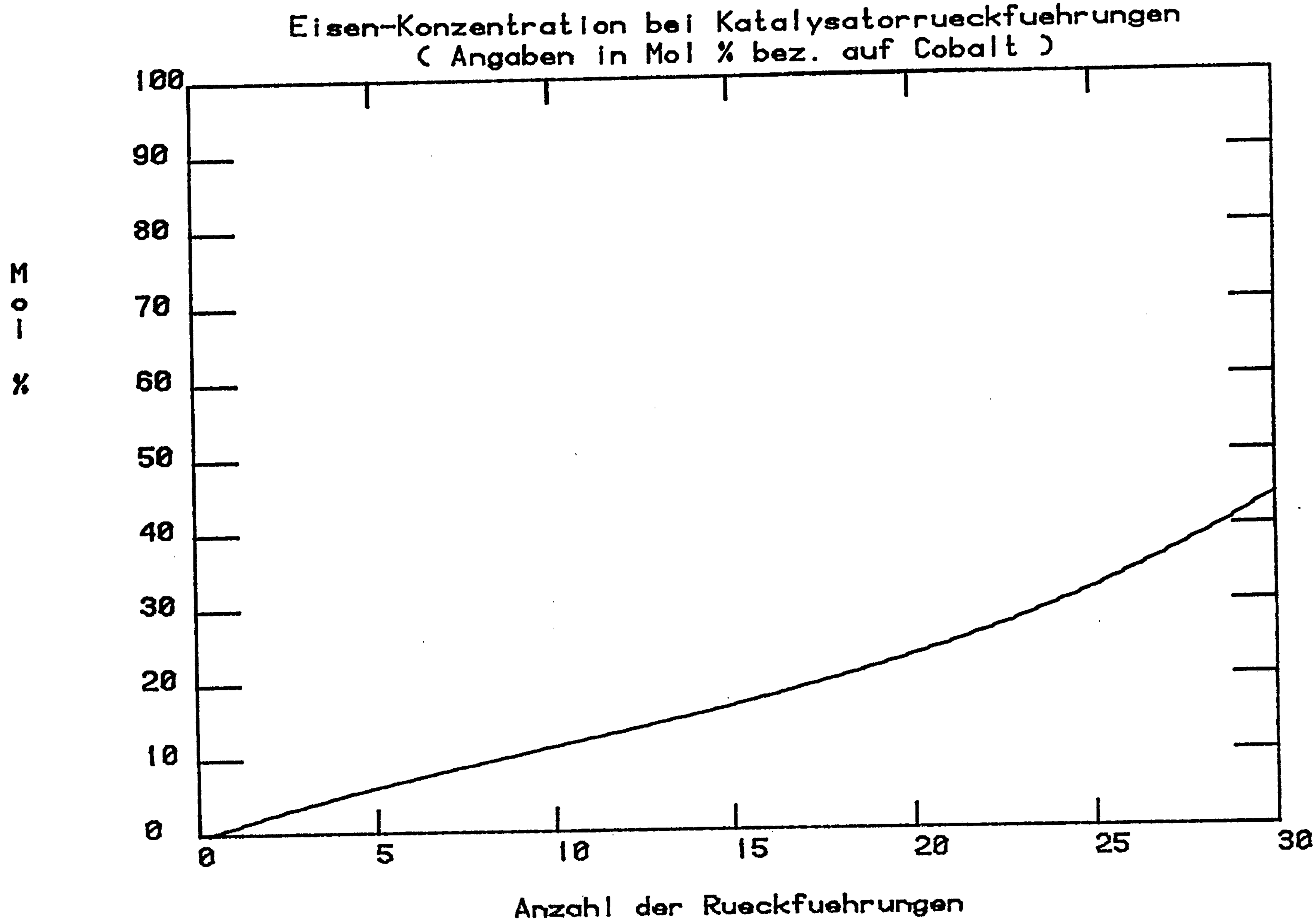
Eisen-Konzentration bei Katalysatorrueckfuehrungen
( Angaben in Mol % bez. auf Cobalt )
Mol %
100
90
80
70
60
50
40
30
20
10
0
0
5
10
15
20
25
30
Anzahl der Rueckfuehrungen